Europäisches Patentamt

European Patent Office

Office européen des brevets

(11) Publication number: **0 152 072**
**A2**

# (12) EUROPEAN PATENT APPLICATION

(21) Application number: **85101319.3**

(22) Date of filing: **08.02.85**

(51) Int. Cl.⁴: **A 61 L 2/08**
**A 61 L 2/10**

(30) Priority: **16.02.84 US 580848**
**14.01.85 US 690451**

(43) Date of publication of application:
**21.08.85 Bulletin 85/34**

(84) Designated Contracting States:
**FR GB IT NL SE**

(71) Applicant: **MOLECULAR BIOPHYSICS TECHNOLOGY, Inc.**
**3508 Market Street**
**Philadelphia Pennsylvania 19104(US)**

(72) Inventor: **Cornelius, Paul A.**
**2901 W. Queen Lane**
**Philadelphia, PA 19129(US)**

(72) Inventor: **Hochstrasser, Robin M.**
**242 Philip Place**
**Philadelphia, PA 19106(US)**

(72) Inventor: **Kallenbach, Neville R.**
**1834 Pine Street**
**Philadelphia, PA 19103(US)**

(72) Inventor: **Rubin, Harvey**
**1925 Manning Street**
**Philadelphia, PA 19103(US)**

(72) Inventor: **Todaro, George J.**
**1940 15th Avenue East**
**Seattle Washington 98112(US)**

(74) Representative: **Königseder, Claudia**
**Zugspitzstrasse 65**
**D-8104 Grainau(DE)**

(54) Pulsed light selective photolysis process for treatment of biological media and products made thereby.

(57) A novel irradiation process and products made thereby are disclosed. Biological media such as blood, blood fractions, genetically engineered protein products and vaccine preparations are treated by said process, photolyzing nucleic acids in preference to proteins in said media. E.g., DNA- or RNA-containing pathogens are inactivated while leaving the proteins substantially intact or functional. In general, the process comprises irradiating the medium with pulsed light of wavelength and flux selected so that (1) the nucleic acids in their ground state absorb radiation and thereby rise to an excited state or states, (2) the nucleic acids in their excited states absorb radiation and thereby rise to higher energy states and undergo photolysis, and (3) the proteins in their ground or their excited states do not absorb sufficient radiation to undergo substantial photolysis. It is surprising and unexpected that nucleic acids in their excited states undergo efficient photolysis whereas proteins under the same conditions in the same medium are kept substantially intact.

Croydon Printing Company Ltd.

1053-001/A

**0152072**

# PULSED LIGHT SELECTIVE PHOTOLYSIS PROCESS FOR TREATMENT OF BIOLOGICAL MEDIA AND PRODUCTS MADE THEREBY

The present invention relates to the field of the 'sterilization' of biological media or fluids such as blood and blood fractions, e.g., blood plasma, blood serum, blood factor VIII,etc., genetically engineered protein products and vaccine preparations. The term 'sterilization', as applied to the present invention, refers to the deliberate alteration, by photolysis, of the chemical structure of nucleic acid entities in the presence of proteins to cause a loss of viability or infectivity of said entities, while substantially maintaining the functionality of those proteins that are also present. An object of this invention is thus to destroy a significant amount or substantially all DNA and RNA based agents, both known and unknown, including infectious nucleic acid molecules capable of transformation, viroids, and viruses or bacteria which are suspected to be in said media, while leaving the proteins in said media to a large degree or substantially completely intact. Until the present invention, it has not been possible to selectively and efficiently photolyze nucleic acids in the presence, and to the substantial exclusion, of proteins. While the process of this invention can be applied to a wide variety of biological media, three illustrative examples are discussed below.

Blood sterilization:

Although various techniques have been proposed for sterilizing blood and blood fractions, at the present time such blood and blood fractions are not effectively sterilized prior to transfusion. As a result, a significant percentage of transfusion recipients contract diseases such as hepatitis, and AIDS, from such transfusions. See New England Journal of Medicine, Vol. 310, pages 69 to 74 (1984).

Techniques which have been suggested but not widely adopted, include passing blood products through bacterial and/or viral filters, adding antibiotics to such products etc. These have not proven reliable or efficacious (filters are difficult to maintain with sufficient flow; adding potentially toxic agents is undesirable).

It is current practice to test transfusates to identify those which contain certain pathogens. However, such tests are not 100% accurate and there are many pathogens for which there is no known test, and such tests may be time-consuming and expensive.

Genetically Engineered Proteinaceous Products of Mammalian Cells:

Most products of recombinant DNA techniques are now produced using altered cells of bacterial strains such as E.coli. While certain products, such as the insulin protein, can be quite effectively produced using such non-mammalian strains, certain other protein products are not easily obtained from such strains due to the inability of those strains to produce the desired protein end product in a complex with other desired molecules such as carbohydrates. Indeed, it has been suggested that many of the desirable proteinaceous end products of genetic engineering techniques may not be efficiently or effectively produced using non-mammalian cell strains.

It has been suggested that mammalian cell lines may be well suited to the genetically engineered manufacture of many protein end products. However, certain problems are anticipated in the use of such cell lines. See "Injectable Monoclonal Antibody Products: Regulatory Concerns" by Bruce Mechant, presented at the Regulatory Affairs Professional Society Meeting, Nov. 9, 1983; "Points to Consider in the Production and Testing of New Drugs and Biologicals Produced by Recombinant DNA Technology", Department of Health and Human Services, Nov. 18, 1983. Mammalian cells are not generally expected to release or secrete their proteinaceous products directly into their surrounding medium. Accordingly, the harvest of such products will probably require rupturing cellular membranes to release those products into a medium from which they may subsequently be refined or purified. Such rupture, however, will also release mammalian DNA and/or RNA into such medium. Particularly because many easily cultured cell lines are mammalian cancer cells, a need exists to ensure active mammalian DNA or RNA is not present as a contaminant in the proteinaceous end product. This need will remain even if other refining or separation processes are used that do not completely eliminate such DNA or RNA. See "Engineering Tomorrows Vaccines" by T. Wilson, Biotechnology 2(1):29-40, January 1984.

Vaccine Preparations:

It is also desired to inactivate viral DNA or RNA during the production of "killed" or attenuated virus vaccines. During the production of such vaccines it is often desired to use techniques to weaken the infectivity of such viruses, while retaining some degree of immunogenic integrity in those viruses.

While the exact mechanisms of viral attenuation and immunogenic retentivity are not known, it is theorized that certain attenuating or killing treatments alter the structures of some or all of the protein coats, nucleic acids, or both of the subject virus such that they are incapable of instituting a serious infection, while nonetheless leaving at least portions of the viral coat, and perhaps even the nucleic acid, intact to act as 'immunogenic site(s)' which serve to stimulate antibody production in response to vaccination.

Various techniques are known for killing or attenuating viruses for vaccine use. These include chemical techniques etc. Although it has been suggested that some viruses are not tolerant of ultraviolet light, it has also been disclosed that ultraviolet light may be used to inactivate viruses without destroying their immunogenic sites. US patent 4,021,364 (microencapsulation of virus possible 'if ultraviolet light is well tolerated') and US patent 4,071,619 (purified and concentrated live vaccine is treated with UV radiation at doses of 5.000 to 200.000 erg/cm² to kill the virus without affecting its immunogenic properties).

Despite these general disclosures, a need exists to improve the selectivity of such inactivations such that protein viral coats will better retain their integrities while nucleic acid kill rates are enhanced.

Ultraviolet Light as a Desinfectant:

It is known that ultraviolet light can be used to sterilize certain materials. Typically, such sterilization is effected through prolonged exposure (i.e., minutes to hours) of such materials to conventional ultraviolet light sources in the 50 to 1000 watt range.

Considerable attention has been given in the past to the effects of UV radiation on biological systems, particularly with respect to the possible mutagenic, cellular, molecular and/or lethal effects of such radiation on bacterial and viral species. It is know, for example, that the DNA of certain species may be inactivated by radiation generally in the 230 to 470 nm wavelength range, and that the sensitivity of the DNA to such radiation is dependent upon the wavelength of that radiation. These effects have been observed using conventional lamps, such as mercury-xenon or vapor lamps. The capability of steady UV irradiation to destroy DNA is noted in "Oxygen-Independent Inactivation of Haemophilus Influenzae Transforming DNA by Monochromatic Radiation: Action Spectrum, Effect of Histidine and Repair", Cabrera Juarez et al, Photochemistry and Photobiology, 23:309-313 (1976).

Molecular Action of Ultraviolet Light:

Scientists are also aware of the behavior of particular organic compounds when irradiated by UV light. It is known, for example, that different organic compounds exhibit different absorption (extinction) coefficients, that is, the ability of each to absorb energy from light varies from compound to compound as well as with the wavelength of that light. It is further understood that absorbed light may raise a given organic molecule from its ground state to a higher energy state, that the molecule will remain in its higher energy state for a very short period of time (known as the 'lifetime' of that energy state), and that said compound may then undergo a chemical reaction leading to the permanent alteration of its structure, or may spontaneously return to the ground state or an intermediate lower energy state. For a general description of the behavior of such organic molecules when irradiated with UV light, see "Some Principles Governing the Luminescence of Organic Molecules" by R.M. Hochstrasser, appearing in Excited States of Biopolymers, edited by Robert F. Steiner, Plenum Publishing Corp. (1983).

Proteins and their constituent amino acids have been studied to determine their behavior in response to UV radiation. Although most of the amino acids of which proteins are comprised do not readily absorb UV light (of wavelengths greater than 220 nm), tryptophan, and to a lesser extent, phenylalanine and tyrosine, are known to absorb significant amounts of UV light, and as a result to be susceptible to structure-altering light-induced chemical reactions, processes herein referred to as "photolysis".

It is known, for example, that the photochemical destruction of tryptophan in an aqueous solution may be induced through irradiation at wavelengths between about 240 and 310 nm, and that the efficiency of that photochemical destruction, expressed in terms of the 'quantum yield' for that destruction, attains its maximum value when that compound is irradiated with light in the 240-250 nm range. See "Ultraviolet Action Spectrum for Tryptophan Destruction in Aqueous Solution" by Raymond F. Borkman, Photochemistry and Photobiology, 26:16-166 (1977).

Certain aspects of the response of tryptophan to irradiation with ultrashort, picosecond length pulses of UV light. have been the objects of several studies. It is known, for example, that when free tryptophan is excited in aqueous solutions its fluorescence lifetime is dependent upon a number of factors such as pH, temperature, etc., and is generally in the 3 nanosecond range.

For tryptophan incorporated in proteins the situation is considerably more complex and remains the subject of much controversy; for instance, the fluorescence lifetime drops into the subnanosecond range in hemoproteins (found in red blood cells). See "Non-Exponential Fluorescence Decay of Aqueous Tryptophan and Two Related Peptides by Picosecond Spectroscopy" by G.R. Fleming et al, Proc. Natl. Acad. Sci. USA, 75:(10) 4652-4656 (1978); and "The Rates of Photodestruction of Tryptophan Residues in Human and Bovine Ocular Lens Proteins" by Borkman et al, Exp. Eye Res., 32:747-754 (1081); and "Fluorescence Lifetimes of Chromophores in Intact Human Lenses and Lens Proteins" by Borkman et al, Exp. Eye Res., 32:313-322 at 314 (1980); and "The Destruction of Tryptophanyl Residues in Trypsin by 280 nm Radiation" by Volkert et al, Photochemistry and Photobiology 17:9-16 (1973).

Even though tryptophan is the least common amino acid of human proteins, the major pathway for UV photodamage (in the 240 to 310 nm range) to proteins involves tryptophan photolysis since it is the most photolabile amino acid. Thus, such radiation may lead to the inactivation of those proteins through photolysis of the tryptophan components, or through photolysis of other aromatic residues. Consequently, while the present invention is concerned with preservation of proteins in general, tryptophan is frequently referred to herein as exemplary.

In 'classical' photochemical reactions, a single photon causes one molecule to undergo a chemical change. However, when molecules in any form of matter are irradiated with sufficiently intense light fields, it is known that other photochemical pathways can be opened up as a result of a single molecule absorbing more than one photon. Many examples of chemical change being induced by more than one photon per molecule exist in the scientific literature of the past 30 years. The best known example concerns the photosynthetic process in green plants, which involves the consecutive absorption of red and yellow photons. Another example concerns molecules having metastable triplet states. The long lived triplet states often become populated subsequent to the absorption of a photon by the molecule. This population of triplets may then absorb another photon having either the same or a different color to result in a chemical process. These concepts formed the basis of a recent monograph by V.S. Letokhov "Nonlinear Laser Chemistry", Springer Verlag New York, (1983).

Such phenomena have been studied using peptide molecules consisting of a chain of amino acids including tryptophan, alanine and glycine. See "Multiple Photon Processes in Molecules Induced by Picosecond UV Laser Pulses", Antonov et al, pages 310-314; "Picosecond Phenomena I", Springer Verlag, New York, 1979. According to this study, when a peptide is irradiated by picosecond pulses, the probability of excited molecules absorbing one or more additional photons is much higher than when nanosecond pulses are used.

The nucleic acid components of DNA and RNA and intact DNA have been the subjects of studies in terms of their response to ultrashort high intensity UV irradiations. These studies have been reviewed by Stanley L. Shapiro in "Ultrafast Techniques Applied to DNA Studies", Biological Events Probed by Ultrafast Laser Spectroscopy, edited by R.R. Alfano, pages 361-383 (Academic Press, New York, 1982). As explained therein, ultrashort light pulses have been proposed to cause the deliberate alteration, or even the destruction, of a complex molecule. Effort has also been directed at achieving selective photochemical reactions in nucleic acids and their components, even though the absorption band of DNA is generally broad, having an absorption peak near 265 nm. See, e.g., "Selective Action on Nucleic Acids Components by Picosecond Light Pulses", Angelov et al, Applied Physics, 21:391-395 (1980). However, because of the non-specific nature of the absorption band of DNA or RNA materials, and because proteins have a similar broad absorption band, selective and efficient photolysis of DNA in preference to proteins in the same media has not been achieved prior to the present invention.

It has been reported that DNA and its components, when exposed to high power ultrashort UV laser action, may successively absorb two UV quanta to thereby acquire energy which exceeds the ionization limit. As a result, some photoproducts are formed which structurally differ from those formed by ordinary 'continuous wave' (cw) UV radiation. It has further been suggested that the photodecomposition efficiency of DNA components under picosecond UV irradiation is more than ten times higher than under nanosecond irradiation and that the process of two-step excitation of molecules under irradiation depends upon many laser radiation parameters such as the radiation wavelength of the first and second steps, the time delay between pulses, the intensity etc. Thus, it is reported that the process of two-step photodecomposition may be made more effective for a desired type of nucleic acid base by choosing laser radiation parameters. For example, it has been reported that viruses may be inactivated using UV irradiation intensities from $10^7$ to $10^9$ watts per square centimeter to cause single-strand breaks in the DNA, whereas the use of lower power UV irradiation is reported to result in inactivation because of the formation of pyrimidine dimers of the cyclobutane type. See "High-Power UV Ultrashort Laser Action
on DNA and its Components" by Angelov et al, Picosecond
Phenomena III, edited by Hochstrasser et al, pages 336 to
339, Springer Verlag New York, 1980.

It has further been reported that multi-step, multiple
photon excitation of atoms and molecules by laser radiation provides a basis for non-linear laser photochemistry.
See Antonov et al, Picosecond Phenomena I, Springer-Verlag
New York, 1979, and "Optical Detection of the Triplet
State of Uracil", D.H. Williams et al, Biochemical and
Biophysical Research Communications, 36(b):912-918,1969.

Further Background Regarding Quantum Mechanics of Nucleic
Acids and Proteins:

The interaction between light from a conventional source
and a sample of molecules can be understood by means of
a few simple physical principles. A source producing
light energy at a single wavelength my be described in
terms of that wavelength and its intensity as expressed
in energy per unit of illuminated areas per unit of time.
In quantum mechanical terms, light energy may be described
using the Planck relationship $E=hv$, where E is the energy
of one photon, h is Planck's constant, and v is the frequency of the light. In this terminology, the light intensity may be expressed in units of photons per cm² per
second, or photons $cm^{-2}$ $sec^{-1}$.

The concentration of sample molecules in a target region
may be expressed in terms of the number of moles per
liter (molarity M). As the light passes through the
sample, it is absorbed at least in part by the molecules,
the efficiency of that absorption being a characteristic
of the particular molecule and varying according to the
wavelength of the light used. The extinction coefficient,
e, is a measure of the absorption efficiency and has the
units $M^{-1}$ $cm^{-1}$. A graph of the extinction coefficient versus wavelength of light is the absorption spectrum
of the molecule.

Quantum mechanically, the absorption of a photon by a
molecule is accompanied by a transition, or change in
the quantum state of the molecule. In a typical case, all
of the molecules may be assumed to occupy their lowest
energy ("ground") quantum state before the light source
is switched on. In the presence of light, some of the
molecules undergo transition from the ground state to
a higher energy state ("excited"). A molecule cannot
remain in such a state indefinitely and must inevitably
lose its excess energy in some way. The fate of any
single molecule cannot be known in advance but only expressed in terms of quantum mechanical probabilities or
quantum yields (Q). An important parameter associated
with each excited state is the lifetime (T), which is
the average amount of time that an undisturbed molecule
occupying that state remains in that state.

There are several possible consequences of exciting a
molecule with light. The molecule may spontaneously return to the ground state, either directly or by first
entering an intermediate state. It might alternatively
absorb another photon to reach an even higher energy state.
Another possibility is that the molecule will undergo
photochemistry and thus suffer an alteration in its
chemical structure; in many cases, the alteration is permanent and the molecule cannot return to its original
ground state. When such photolysis is used to cause a
chemical change in a DNA or protein molecule, the biological functioning of the molecule may be impaired or
destroyed.

Absorption coefficients, excited state lifetimes, and
quantum yields are intrinsic molecular characteristics
and, as discussed above, have been determined through experimentation for many molecules. From a knowledge of
these values and the parameters of the light source one
can calculate the rate of a photochemical reaction.

The present invention is concerned with irradiation of a medium which comprises nucleic acids, e.g., in the form of DNA molecules, and proteins. The following calculation demonstrates that the consequence of irradiating the medium with a conventional source of UV light, such as a discharge lamp, is the destruction of the proteins as well as the DNA, since both chemicals absorb the light and are destroyed by the subsequent photolysis. The calculations below are based upon the Beer-Lambert absorption law, which defines the radiative transition rate per molecule (r) as the product of the absorption cross-section and the light intensity:

$$r = I \times s$$

where I is the light intensity in photons $cm^{-2}$ $sec^{-1}$ and s is the absorption cross-section in $cm^2$. The absorption cross-section s is obtained from the extinction coefficient by the formula $s = 3,82 \times 10^{-21} \times e$, where the extinction coefficient is in units of $M^{-1} cm^{-1}$. s has the units of $cm^2$. The overall rate per molecule (R) of the photochemical reaction is the product of the transition rate and the photochemical quantum yield:

$$R = I \times s \times Q$$

From a knowledge of R one can compute the probability, (P), that a given molecule will remain unreacted during a time interval of t seconds:

$$P = 10^{-(R \times t)/2,303}$$

In this calculation, the attenuation of the light beam as it passes through the sample medium is neglected. This approximation is valid assuming that the subject sample meets the thin layer criterion discussed herein, that is, that the sample is sufficiently thin or the absorption coefficient sufficiently small so that the attenuation of the light beam through the sample medium will be small. Human blood plasma, for example, contains a number of different protein molecules. Most amino acids (the individual units from which proteins are constructed) do not significantly absorb near-UV or mid-UV light and are not

0152072

affected by the presence of such light. Tryptophan, discussed above, is a notable exception. A typical plasma protein contains only a few (0 to 15) tryptophans, but even a small number is sufficient to make a protein susceptible to photochemical damage. See Atlas of Protein Sequence and Structure, Vol. 5, Supplement 3, M.O. Dayhoff ed. (National Biomedical Research Foundation, Silver Spring, MD., 1976). As discussed above, the extinction coefficient and photochemical quantum yield have been measured for tryptophan, thus making it possible to estimate the rate at which a given light source will damage a given protein. See Handbook of Biochemistry and Molecular Biology, 3rd Edition, Vo. 2 (Chemical Rubber Co., Cleveland, 1976); G.R. Fleming et al, Chemistry , 75:4652 (1978) and references cited therein; and Raymond F. Borkman, Photochemistry and Photobiology, 26:163 (1977) supra.

If blood plasma is taken from a donor with a viral infection, the plasma may contain as many as one million viruses per cubic centimeter. A virus consists of one or more DNA (or RNA) molecules inside a coat, typically a protein or protein-lipid complex. Other infectious agents called viroids, which consist only of an RNA molecule, are also known. The viral coat is relatively transparent to UV light, but the nucleotide bases of DNA (or RNA) are all strong absorbers. A sufficient dose of UV light will create photochemical lesions in the viral DNA, resulting in an irreversible loss of infectivity. Many experiments have measured the rate at which UV light deactivates viruses under various conditions. These experimental data effectively define a quantum yield for viral inactivation, permitting a comparison between the efficiency of viral inactivation and the efficiency of protein damage for a given light source. See Photochemistry and Photobiology, 26:163 (1977) supra.

TABLE I

Extinction coefficients and absorption cross-sections
at a wavelength of 266 nm

| Species | $e(M^{-1}cm^{-1})$ | $s(10^{-17}cm^2)$ |
|---|---|---|
| Tryptophan | 4.000 | 1,5 |
| Nucleic acid (average) | 10.000 | 3,8 |
| Viral DNA molecule | $1 \times 10^9$ | $3,8 \times 10^5$ |
| Model plasma protein | 40.000 | 15,0 |

Notes: The above figures assume a protein having 10 tryptophans and a viral DNA molecule containing 50.000 nucleic acid base pairs. There are four different bases in DNA and the above extinction coefficient represents an approximate average (the four bases have very similar absorption spectra). Some viruses contain RNA instead of DNA; the spectral properties of the two polymers are similar. There are two other amino acids (tyrosine and phenylalanine) that have significant UV absorption. Their extinction coefficients at 266 nm are 700 and 100, respectively.

Concentration of viruses in an infected human ca. $10^6$/ml
Concentration of proteins in human plasma ca. $10^{-3}$M
Radiation flux (the product of the light intensity and the irradiation time, i.e., I x t) at 254 nm necessary to reduce viral DNA activity by 90 % $1,3 \times 10^{17}$ photons/cm²
Equivalent 'quantum yield' for viral destruction $2 \times 10^{-6}$

Table I presents the quantum yield and cross-sections of both a model virus and a model tryptophan-containing protein in the near UV part of the spectrum. In addition, since a unit (450 ml) of infected blood may contain as many as $4,5 \times 10^8$ viruses, sterilization should preferably reduce the viral activity by a factor of, appr., at least $10^8$. In other words, the probability of an individual virus remaining unreacted (P) should be less than $10^{-8}$. From these data it has been calculated that a continuous wave light source operating near 260 nm would be required to supply a total radiation flux of $2 \times 10^{18}$ photons per cm² in order to achieve the desired condition (a probability of unreacted viruses of less than $10^{-8}$) of viral deactivation. It follows that this amount of radiation will also result in massive protein destruction (P = $10^{-11}$ for the model protein of Table I). Thus, such a light source is not capable of sterilizing such biological fluids in a useful way.

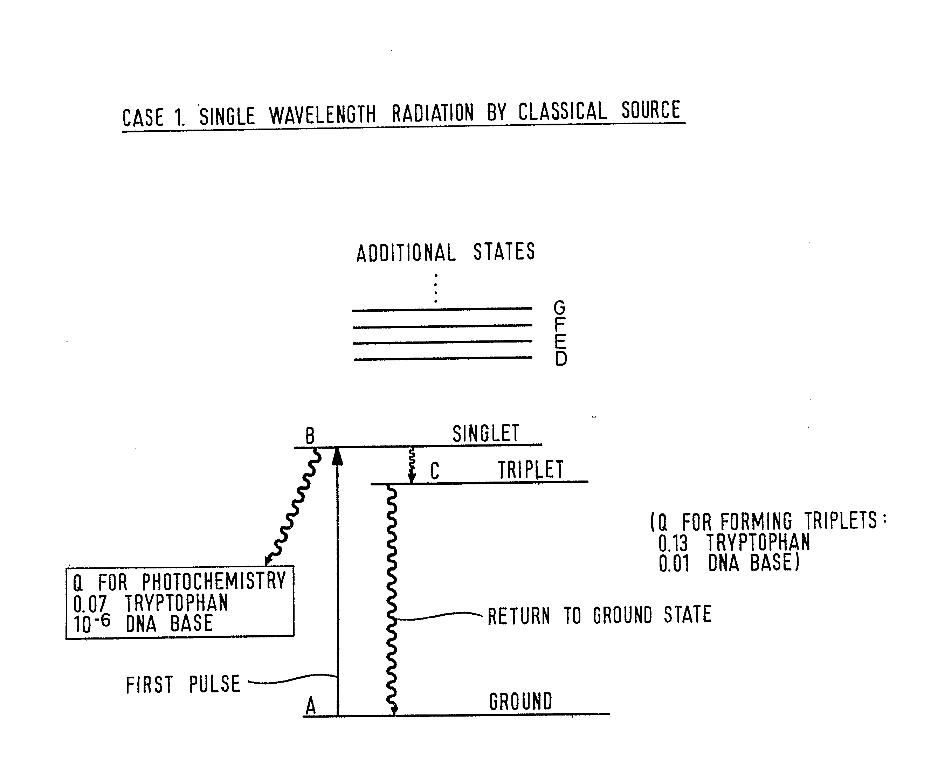

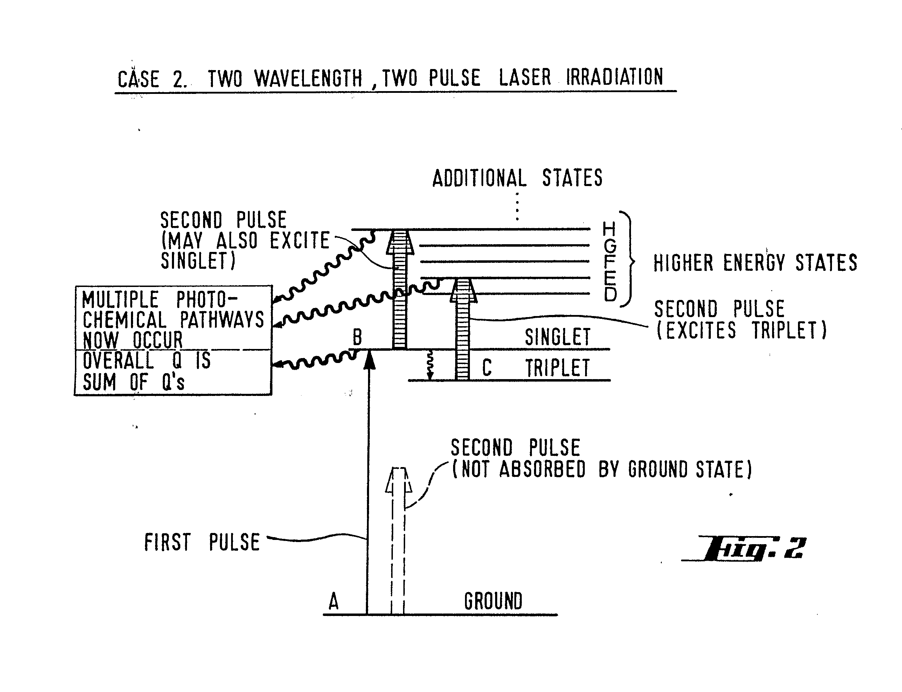

It should also be noted that the Beer-Lambert law does not always provide a correct descriptiom of the rate of a photochemical process, since it can only be applied in cases where the intensity of the light source is kept relatively low. Modern pulsed lasers are capable of producing picosecond ($10^{-12}$ sec.) bursts of extremely high intensity. A single pulse from such a laser can attain a power in excess of 1 gigawatt ($10^9$ watts), while continuous wave lasers or 'classical' sources (such as discharge lamps) typically operate in a range of 10 to 1000 watts. The effects of such intense pulses on the molecules of the sample can be understood by comparing the differences between the processes of Figures 1 and 2.

When the intensity of incident photons is relatively low, there is only a small probability that a molecule will absorb a second photon while in an excited state. Thus, as seen in Fig. 1, there is a high probability that after a single photon has raised the energy level from ground state A to excited state B, the molecule will spontaneously undergo photolysis, form a lower energy state and/or return to the ground state without absorbing another photon. In a picosecond pulse, on the other hand, the photon intensity is quite high. In this case, there is a significant probability that a second photon can be absorbed while the molecule is still excited, and that in this way the molecule can reach energy levels that are inaccessible by simple one-photon Beer-Lambert type processes.

Other workers have recently applied some of the quantum mechanical concepts discussed above to inactivation of free nucleotides. See A. Andreoni et al, "Two-Step Laser Photobiology: Application For Cancer Treatment", 13th International Quantum Electronics Conference, pp.142; A. Anders "Laser Fluorescence Spectroscopy of Biomole- cules: Nucleic Acids", Optical Engineering, 22(5), pages 592-595 (1083). None, however, has accomplished the photolysis of DNA or RNA nucleic acids in preference to and in the presence of proteins, i.e., an efficient sterilization process, as provided by the present invention.

The achievement and advantages of the present invention can be further appreciated by reference to US patent 4 395 397, 3 837 373, 3 941 670, 3 817 703, 3 955 921, 4 042 325, and 4 265 747, none of which teaches a selective and efficient photolysis of nucleic acids in biological media while leaving proteins and other biological materials in the media undisturbed.

US patent 4 395 397, in particular, is illustrative. There, it was described to kill unwanted cells, e.g., cancer cells, in a suspension of living cells. A process was disclosed whereby the cancer cells were first identified or 'tagged' by means of fluorescent antibodies and the cells so tagged were then killed, one cell at a time, with laser light. In contrast, and as will be apparent from the description below, the present invention does not require identification of the unwanted nucleic acids prior to treatment of the biological medium desired to be sterilized. Indeed, the process of the present invention can be used to provide sterilized products, e.g., blood, without first determining whether or which nucleic acid- based pathogens, e.g., cancer cells or viruses, are present. Moreover, the present invention does not require use of a 'tag' and does not require response to a fluorescent or other 'signal'.

The present invention provides a process for treating a biological medium to enhance photolysis of nucleic acids relative to proteins present therein. The invention also Provides products made by said process. The process comprises irradiating the medium with pulsed light of wavelength and flux selected so that (1) the nucleic acids in their ground state absorb radiation and thereby rise to an excited state or states, (2) the nucleic acids in their excited states absorb radiation and thereby rise to higher energy states and undergo photolysis, and (3) the proteins in their ground or their excited states do not absorb sufficient radiation to undergo substantial photolysis.

By application of the process, the medium is sterilized, e.g., nucleic acid or viral activity is reduced by at least $10^4$ while protein functionality is reduced by no more than 40%.

Such results have not been achieved prior to the present invention and it is surprising and unexpected that nucleic acids in their excited states can be made to undergo efficient photolysis while proteins under the same conditions in the same medium can be kept substantially intact. Preferably, the biological medium is a solution selected from blood, blood fractions, genetically engineered proteinaceous products of mammalian cells, and vaccine preparations containing viral DNA or RNA in a protein coat. All of these media share the common characteristic of containing nucleic acids (e.g., in the form of DNA or RNA) and proteins.

Thus, in accordance with certain embodiments of the present invention, a solution of proteins, e.g., tryptophan-containing proteins, and nucleic acids is treated to selectively photolyze or inactivate those nucleic acids. These embodiments comprise irradiating that solution with a first light pulse of a first wavelength and of sufficient flux to raise a portion of those nucleic acids from their ground state to one of their excited states, and of further irradiating those nucleic acids in those excited states with a second light pulse which is preferably absorbed by nucleic acids in those excited states to raise said nucleic acids to even higher energy states from which the spontaneous photolysis of said nucleic acids occurs. In performing these embodiments, the flux and wavelengths of the first and second light pulses are carefully selected to minimize the photolysis of the amino acids, such as tryptophan, of said proteins. This can be accomplished, for example, by the selection of pulses that are of substantially the same wavelength and flux, or by the selection of pulses that are of substantially the same wavelength and different flux, or, by the selection of pulses that are of different wavelength and flux. The pulses can be repeated and/or sequenced, if desired, in various ways as explained in more detail below. In general terms, therefore, the present invention relates to the use of a sequence of light pulses suitably arranged by time and wavelength in order to control the outcome of a photolysis, e.g., to selectively react DNA or RNA molecules in the presence of proteins.

Accordingly, it is an object of the invention to provide an irradiation process for sterilizing biological media.

It is another object to provide an irradiation process for sterilizing a biological medium selected from blood, blood fractions, genetically engineered proteinaceous products of mammalian cells, and vaccine preparations.

It is another object of the invention to provide a novel irradiation process for treating biological media to photolyze nucleic acids in preference to proteins present therein.

It is another object of the invention to provide a process which utilizes a sequence of light pulses suitably arranged by time, wavelength and intensity, to control the outcome of a photolysis, e.g., to enhance the difference in the relative rates of photolysis as between nucleic acids and proteins.

It is another object of the invention to provide treated biological media, e.g., media in which the nucleic acids have been photolyzed in preference to proteins present therein.

It is another object of the invention to produce, by the process of the invention, nucleic acid inactive, protein-rich end products, e.g., sterilized proteinaceous end products of mammalian cell origin, killed virus vaccines, blood and blood fractions.

Fig. 1 is a diagram illustrating certain energy states of DNA and tryptophan as well as certain photochemical processes that result from irradiating these compounds with a classical light source which predominantly results in single photon photochemistry.

Fig. 2 is a diagram similar to Fig. 1 illustrating the additional photochemical processes of tryptophan and DNA induced by a preferred ultrashort two-pulse differential wavelength laser irradiation process of the present invention.

A preferred embodiment of the present invention involves flowing a thin layer of the biological medium, in the form of a fluid, through a target region which is disposed to receive the output pulses of a light source, e.g, a laser. As used herein, the term "thin layer" refers to a layer of fluid which transmits more than 10% of the light energy which is incident thereon. Depending on the nature of the medium and its possible dilution, layers satisfying this criteria are expected typically to have thicknesses of from 0,1 to several mm, preferably thicknesses on the order of less than 0,5 mm, and most preferably about 0,2 mm. The actual flow rate of the fluid through the target region depends upon the effective area of the incident laser beams and the intensities and repetition rates of the pulses, as described hereinafter.

It is anticipated that in most installations the flow across each mm of target region width can be established at about 5 ml per sec. through a quartz channel which defines a layer of generally square or rectangular cross-section and which presents as part of its largest surface an area equal in width to or slightly narrower than the width of the incident light beam.

The pulsed light used in this invention is preferably comprised of laser pulses. Pulsed laser systems produce their output pulses in a repetitive fashion. The rate at which the pulses are produced is dependent on the laser hardware and is called the 'repetition rate'. In the treatment of the biological medium the pulses are preferably directed onto a small spot (either circular or of some other shape) referred to as the 'target region'. This region will, in most instances, be too small to contain the entire sample to be processed. In these instances, the sample can be flowed through the target region until the entire sample has been irradiated.

Alternatively, the laser beam can be caused to scan over the area of the sample and/or the sample can be processed as a sequence of sub-samples. To insure that each volume element of the sample is subjected to substantially the same irradiation conditions, each volume element should receive repetitions of the same cycle of laser pulses.

In a specific embodiment of the invention, blood plasma or blood serum can be sterilized in the presence of intact blood cells. A red blood cell contains no DNA and is relatively resistant to radiation at the wavelengths and fluxes described herein. However, a single red blood cell has sufficient optical density to absorb the bulk of the incident radiation, thereby shielding the portion located behind said blood cell. Accordingly, when whole blood is to be processed it is preferred to establish a thin channel flow of blood through the target region such that the cells will pass "single file" through that region. The plasma or serum surrounding these cells is then irradiated from opposing directions to insure that the entirety of the subject plasma receive the desired amount of UV radiation.

The quantum yield of a two-photon photochemical process as is involved in the present invention depends upon the intensity of the incident light. For a typical laser system, the energy content and the time duration of the output pulse are initially determined by the laser hardware. The intensity of the laser light at the target region can be brought to virtually any desired value, however, by passing the laser pulse through a lens (or set of lenses) to control the cross-sectional area of the pulse as it enters the target region. For this reason, a wide variety of existing laser systems can be used to practice the present invention, and the choice of a particular laser is largely dictated by cost, reliability, and the processing rate desired.

Pulsed lasers are currently available with repetition rates ranging from 0,01 Hz (pulses per second) to $10^8$ Hz. Those lasers having high repetition rates typically produce pulses that are weak; such pulses should be focussed to a very small spot to generate sufficient intensity to carry out the present process. Lasers with very low repetition rates typically produce pulses of enormous energy, but presently suffer from poor reliability. To provide both adequate peak power (e.g., to stimulate the two-photon absorption process described herein) and adequate average power (e.g., to treat sufficient volume of material), a preferred laser for the present invention (A) has a repetition rate betweem 10 Hz and 1.000.000 Hz, more preferably between 10 and 10.000 Hz, and most preferably between 100 and 10.000 Hz and (B) is capable of producing pulses of durations less than about $2 \times 10^{-8}$ seconds, and preferably $1 \times 10^{-10}$ to $1 \times 10^{-12}$ seconds, while emitting light of extremely high intensity. This laser may be a conventional laser, such as a YAG laser (and its associated optical components), which is capable of providing the wavelength(s), intensities, and pulse frequencies described herein.

0152072

Pulsed lasers typically operate at a single fixed wavelength. Numerous methods are known in the art for generating additional wavelengths from this original pulse, including harmonic generation, synchronous dye laser operation, and optical parametric oscillation. In those embodiments of the invention which uses pulses of different wavelengths, those pulses can be derived from a single original pulse using one of the methods known to the prior art.

Considerations of target region size, sample processing rate, and laser repetition rate apply equally to all of the embodiments of the invention described herein. While the embodiments are described in terms of pulse duration and flux, those skilled in the art can relate these variables to intensity, and to target sizes and sample processing rates by the choice of particular laser systems. The present invention recognizes the importance of maintaining the integrity of blood plasma or serum proteins, while performing DNA or RNA inactivation. Even though blood serum proteins are also susceptible to non-linear inactivation, the present invention recognizes that such proteins can be preserved substantially intact by carefully selecting the wavelengths and intensities of the first and second pulses to favor the photolysis of nucleic acids. For example, this process can increase the efficiency of tryptophan photolysis by a factor calculated to be only about 3 or below while simultaneously increasing the efficiency of DNA photolysis by a factor of about 5.000. It should be understood that the enhancement of the difference in the relative rates of photolysis as between nucleic acids and proteins achieved by the process of this invention is of paramount importance, and that the numbers "3" and "5.000" are calculated for the purpose of illustration and may not be applicable in every case.

As an example of the process of the invention, using a pulsed laser, it is now possible to conduct a two-pulse photolysis, as shown schematically in Fig. 2. The first pulse is chosen to have a relatively low intensity so as to excite a fraction of the molecules to state B by a simple Beer-Lambert process. Of these excited molecules, approximately 13% in the case of tryptophan and 1% in the case of a nucleic acid, will reach state C through the occurrence of intrinsic molecular processes. A second pulse of light of extremely high intensity is now used to cause further absorption events to take place. The existence of higher energy states such as D, E and F has also been demonstrated experimentally and these states are expected to provide a high probability of photochemical reaction. See D.H. William et al, supra, and D.V. Bent at el, Journal of the American Chemical Society 97:2612 (1975). It is important to note that the ground state A cannot absorb photons efficiently from the second pulse, since there is no quantum state available of the appropriate energy.

The consequence of this two-pulse irradiation process is that virtually every molecule that reaches the triplet state C will be forced to react photochemically under the influence of the second pulse. The overall rate of the photochemical reaction depends, in this case, on the rate of producing triplets; this will increase the efficiency of tryptophan photolysis by a factor of 3 but will simultaneously increase the efficiency of DNA photolysis by a factor of 5.000. The effect of the two-pulse scheme is illustrated in Fig. 2. In this example, the sterilization condition (P less than $10^{-8}$) can now be achieved using a 260 nm flux of only $4,6 \times 10^{14}$ photons per cm². This flux will yield a P value for the proteins of 0,99; i.e., about 99% of the protein functionality of the material will be retained.

The present invention thus provides, in one embodiment, novel processes for sterilizing biological fluids such as human blood and blood fractions, which processes involve the use of intense pulses of laser light to destroy infectious agents while maintaining high functional levels

- 23 -

of proteins and other vital components.

In an illustrative general embodiment, the invention provides a process for treating a solution of proteins and nucleic acids such as DNA or RNA to selectively inactivate said nucleic acids, comprising the steps of (a) irradiating said solution with a first light pulse of a first wavelength of sufficient flux to raise a portion of said nucleic acids from their ground state to an excited state yet not sufficient to inactivate the proteins in said solution; and (b) irradiating said nucleic acids while in said excited state with a second light pulse which is preferentially absorbed by nucleic acids in said excited state but not substantially by proteins in their ground or their excited states to raise said nucleic acids to energy states higher than said excited acids state to thereby cause photolysis of said nucleic acids while minimizing the photolysis of said proteins.

Illustrative conditions for this embodiment are as follows: Said excited state comprises nucleic acid in its singlet or triplet state and said second pulse is applied during the singlet or triplet lifetime of said portion of said nucleic acids; said second light pulse is applied within one picosecond after said first light pulse or said first and second pulses are simultaneously applied; the wavelength of said first pulse is between 220 and 280 nm; the duration of said first pulse is less than $2 \times 10^{-8}$ seconds, preferably between about $1 \times 10^{-12}$ and $9 \times 10^{-10}$ seconds; said first pulse has a flux of less than about $5 \times 10^{14}$ photons/cm², preferably a flux of from about $1 \times 10^{13}$ to $5 \times 10^{14}$ photons/cm², and more preferably of from about $1 \times 10^{14}$ to $5 \times 10^{14}$ photons/cm²; said second pulse has a wavelength above about 350 nm, preferably of about 350 to 410 nm or between about 500 to 560 nm; said second pulse has a duration of less than $2 \times 10^{-8}$ seconds, preferably of between about $9 \times 10^{-10}$ to $1 \times 10^{-12}$ seconds; said second pulse has a flux of about $1 \times 10^{15}$ to $1 \times 10^{18}$ photons/cm², preferably of $1 \times 10^{17}$ photons/cm²; said

- 24 -

0152072

light pulses are pulses of laser light; said light pulses are applied by a single laser; said solution is located as a thin layer in a target region, said layer having a thickness of less than about 0,5 mm, preferably of about 0,2 mm; said solution is flowed across each millimeter of target region width at a rate of about 5 ml/sec.; said solution is a blood fraction comprising plasma proteins; said blood fraction further comprises blood cells; and said pulses are applied from a plurality of directions to strike substantially all of the plasma and serum disposed around said blood cells.

It can be seen, therefore, that unique sterilization and protein production processes are provided by the present ivention. These processes use pulses of intense laser light to selectively photolyze DNA in the presence of proteins, e.g., tryptophan-containing proteins.

In certain embodiments, this selectivity is achieved by the use of a sequence of pulses whose wavelength, time duration, and time spacing are under the control of the laser operator. The properties of the secondary pulse or pulses are chosen such that only those molecules that absorbed light from an earlier pulse in the sequence are affected. For this reason, the secondary pulse or pulses can be of extremely high intensity without causing unwanted reactions.

In another illustrative embodiment of the invention, the process is used to treat blood fractions, including blood plasma, blood serum or products thereof, which fractions are suspected of carrying viable or infectious nucleic acid-containing agents. The embodiment, for example, can comprise irradiating a target region of the fraction with ultrashort, multiple light pulses of different wavelengths and intensities. A first pulse (or pulses) having a wavelength(s) of between 220 and 280 nm is applied to achieve a flux in the blood fraction target region of slightly less than $5 \times 10^{14}$ photons/cm². The first pulse or pulses excite(s) the DNA or RNA in said fractions from their

- 25 -

0152072

ground state to excited state(s). A second higher intensity pulse (or pulses) having a wavelength(s) above about 300 nm and a flux of between about $1 \times 10^{15}$ and $1 \times 10^{18}$ photons/cm² is then applied within the excited state lifetime (e.g., up to about 6 microseconds) of said DNA or RNA. As a result, these nucleic acid-containing molecules are excited to an even higher energy state through a nonlinear process, which higher energy state results in their substantial inactivation by photolysis.

In further illustrative particular embodiments, the sterilization processes of the invention can be practiced by using first and second single light pulses which are either simultaneously applied or which are applied, for example, within one triplet state lifetime (approximately 1 microsecond) of each other. As seen from the above specific example of Fig. 2, the first light pulse can be of a wavelength absorbed by nucleic acids and raises a portion of the nucleic acids from their ground state to their triplet state. The second light pulse can be of a higher intensity and longer wavelength which is preferentially absorbed by the nucleic acids in their triplet states and raises those nucleic acids to higher energy states to thereby increase the probability that spontaneous photolysis of such nucleic acids will occur. These first and second light pulses are selected such that the photolysis of the amino acids of the proteins is minimized, e.g., maintained to less than about 1% of the proteins present in the sample. In accordance with this embodiment, the first pulse is less than $2 \times 10^{-8}$ seconds in duration, preferably about $10^{-10}$ to $10^{-12}$ seconds, and has a flux of $1 \times 10^{13}$ to $1 \times 10^{16}$, preferably less than $5 \times 10^{14}$, photons/cm².

Although lower first pulse fluxes will lessen the effect of the subject radiation upon the proteins, a corresponding diminution in the number of excited DNA or RNA molecules will also result. Accordingly, the first pulse flux is preferred to be in the range of betweem $1 \times 10^{13}$ and

$5 \times 10^{14}$ photons/cm², more preferably between $1 \times 10^{14}$ and $5 \times 10^{14}$ photons/cm². The second pulse preferably has a wavelength above about 350 nm, and is preferably within wavelength ranges of either 350 to 410 nm or 500 to 560 nm. It is preferred that the second pulse have a duration of less than $2 \times 10^{-8}$ seconds, preferably about $10^{-10}$ to about $10^{-12}$ seconds. In order to increase the probability of photolysis of the excited DNA triplets, each second pulse has a higher intensity than the first pulse, having a flux of about $1 \times 10^{15}$ to $1 \times 10^{18}$, preferably of about $1 \times 10^{17}$ photons/cm².

For purposes of simplicity, the above discussion has illustratively referred in some instances to the administration of single first and second pulses and to the intermediate state as a triplet state. In other embodiments, the process can be efficiently operated by applying a repetition of pulses having the same wavelength, or a repetition of an alternating series of first and second pulses (e.g., of different wavelength) to the subject sample as it flows through a target area. Also, other excited states (e.g., the first excited singlet) can also be utilized as intermediates.

In accordance with such an embodiment, a target region of a thin layer of a blood fraction or other biological media to be sterilized is irradiated with more than one (e.g., a repetition) of first light pulses comprising a wavelength or wavelengths within a first range of 220 to 280 nm. Each of the first light pulses has a duration of less than $2 \times 10^{-8}$ seconds, and together these first light pulses have a combined flux within the stated wavelength range of between wbout 1 $\times 10^{13}$ to $1 \times 10^{16}$ photons per cm². In accordance with this embodiment, second higher intensity light pulses are repetitively applied simultaneously or sequentially up to no more than 1 microsecond, preferably 1 picosecond, after each of said first light pulses. These second higher intensity light pulses each have a wavelength or wavelengths within a second wavelength range above about 350 nm, each have durations of less than $2 \times 10^{-8}$ seconds, and together have a combined flux within the stated wavelength range of between about $1 \times 10^{15}$ to $1 \times 10^{18}$ photons/cm². Once again, the preferred second wavelength ranges are between 350 and 410 nm or 500 and 560 nm. In accordance with this embodiment, said first light pulses should be applied at a frequency of between 10 and 1.000.000 pulses/second. When this embodiment of the process, as well as others, is applied to a biological fluid that is flowed as a thin layer through a target region of a laser, the frequency of the laser pulses and the selected flow rate of the fluid to be sterilized should preferably be selected such that the fluid to be treated is exposed to the aforementioned combined fluxes prior to leaving the target region.

In the embodiment where pulses of the same wavelength are utilized, the wavelength is preferably within the range of 180 to 295 nm, more preferably of 220 to 290 nm, and most preferably of 220 to 290 nm. The duration of each pulse is preferably less than $1 \times 10^{-5}$ seconds, more preferably less than $1 \times 10^{-8}$ seconds, more preferably in the range of $5 \times 10^{-9}$ to $1 \times 10^{-12}$ seconds, and most preferably in the range of $1 \times 10^{-10}$ to $1 \times 10^{-12}$ seconds. When a duration of from $1 \times 10^{-5}$ to $1 \times 10^{-10}$ seconds is utilized, it is believed that the triplet state comprises the intermediate pathway. When a duration of from $1 \times 10^{-10}$ to $1 \times 10^{-14}$ seconds is utilized, it is believed that the singlet state comprises the intermediate pathway. It is preferred when utilizing pulses of the same wavelength to select conditions which favor the singlet state pathway, i.e., pulses having a duration within the above stated range. Also, in this embodiment where pulses of the same wavelength are utilized, the pulses preferably each have a flux greater than $1 \times 10^{15}$, more preferably from about $1 \times 10^{15}$ to about $1 \times 10^{18}$, more preferably about $1 \times 10^{17}$ to about $1 \times 10^{18}$, and most preferably about $1 \times 10^{17}$, photons/cm². The combined flux of the pulses is preferably about one order of magnitude higher than the flux of each pulse, i.e., it is preferred that about 10 repetitions of each pulse per unit volume of the media be utilized.

When pulses of different wavelength are utilized, the duration of the first pulses is preferably as stated immediately above. The first pulses preferably have a wavelength within the range of 180 to 350 nm, more preferably 180 to 295 nm, more preferably 220 to 290 nm, and most preferably 220 to 280 nm. The first pulses preferably each have a flux of less than $1 \times 10^{18}$, more preferably less than $5 \times 10^{14}$, more preferably $1 \times 10^{13}$ to $5 \times 10^{14}$, and most preferably $1 \times 10^{14}$ to $5 \times 10^{14}$, photons/cm². The combined flux of the first pulses is preferably from $1 \times 10^{13}$ to $1 \times 10^{18}$, more preferably from $1 \times 10^{13}$ to $1 \times 10^{16}$, and most preferably from $1 \times 10^{14}$ to $5 \times 10^{14}$, photons/cm². The second pulses preferably each have a flux greater than $1 \times 10^{15}$, more preferably from $1 \times 10^{15}$ to $1 \times 10^{18}$, and most preferably about $1 \times 10^{17}$, photons/cm². The combined flux of the pulses is, for the reason stated above, about one order of magnitude higher than the flux of each pulse. The preferred wavelength for the second pulses depends upon the duration selected for the first pulses.

Thus, when a duration for the first pulses favoring the triplet state pathway is utilized ($1 \times 10^{-5}$ to $1 \times 10^{-10}$ seconds), the second pulses preferably each have a wavelength greater than 300 nm, preferably in the range of 300 to 700 nm, more preferably 300 to 450 nm, and most preferably 350 to 410 nm. In this embodiment, it is preferred that the duration of each second pulse is less than $1 \times 10^{-5}$, more preferably less than $1 \times 10^{-6}$, more preferably less than $1 \times 10^{-8}$ seconds, preferably in the range of $5 \times 10^{-9}$ to $1 \times 10^{-12}$ seconds, and more preferably in the range of $1 \times 10^{-10}$ to $1 \times 10^{-12}$ seconds. Each second pulse is preferably applied within $1 \times 10^{-6}$ seconds of each first pulse, and more preferably is applied substantially simultaneously with each first pulse. When a duration for the first pulses favoring the singlet state pathway is utilized ($1 \times 10^{-10}$ to $1 \times 10^{-14}$ sec.), the second pulses preferably each have a wavelength greater than 300 nm, preferably in the range of 300 to 700 nm, more preferably 500 to 560 nm, and most preferably about 520 to 540 nm. In this embodiment, the duration of each second pulse preferably is in the range of $1 \times 10^{-10}$ to $1 \times 10^{-12}$ seconds, more preferably less than $3 \times 10^{-11}$ seconds, more preferably less than about $3 \times 10^{-12}$ seconds, and most preferably is in the range of $1 \times 10^{-11}$ to $1 \times 10^{-12}$ seconds. Each second pulse is preferably applied within $3 \times 10^{-12}$ seconds of the first pulse, more preferably is applied substantially simultaneously with the first pulse, and most preferably is applied at a time delay with respect to the first pulse of about $1 \times 10^{-12}$ seconds.

It will be apparent, therefore, that in some of its broader aspects, the present invention can be described as a process for treating a biological fluid containing nucleic acids and proteins, which process comprises irradiating said fluid with a plurality of light pulses of wavelength and intensity selected so that said nucleic acids are photolyzed in preference to said proteins. As noted above, in certain embodiments, these pulses can be laser pulses of the same wavelength, or can comprise first and second laser pulses which have different wavelengths, respectively. When pulses of the same wavelength are selected, preferred conditions are as follows: each of said pulses has substantially the same wavelength within the range of 180 to 285 nm, preferably 220 to 290 nm, more preferably 220 to 280 nm, a duration less than $1 \times 10^{-5}$ seconds, preferably a duration in the range from $5 \times 10^{-9}$ to $1 \times 10^{-12}$ seconds, more preferably from $1 \times 10^{-10}$ to $1 \times 10^{-12}$ seconds, and a flux greater than $1 \times 10^{15}$, preferably in the range from $1 \times 10^{15}$ to $1 \times 10^{18}$, more preferably $1 \times 10^{17}$ to $1 \times 10^{18}$, photons/cm².

When pulses of different wavelength are selected, preferred conditions are as follows: each of said first pulses has a wavelength within the range of 180 to 350 nm, a duration of less than $1 \times 10^{-5}$ seconds, and a flux less than $1 \times 10^{18}$ photons/cm², and each of said second pulses has a wavelength within the range of 300 to 700 nm, a duration of less than $1 \times 10^{-5}$ seconds, and a flux of greater than $1 \times 10^{15}$ photons/cm².

Further preferred conditions when pulses of different wavelengths are selected are as follows: each of said first pulses has a wavelength within the range of 180 to 295 nm, a duration of from $1 \times 10^{-10}$ to $1 \times 10^{-14}$ seconds, and a flux of $1 \times 10^{13}$ to $5 \times 10^{14}$ photons/cm², and each of said second pulses has a wavelength within the range of 500 to 560 nm, a duration in the range of $1 \times 10^{-10}$ to $1 \times 10^{-12}$ seconds, a flux of $1 \times 10^{15}$ to $1 \times 10^{18}$ photons/cm², and each second pulse is applied within $3 \times 10^{-12}$ seconds of each first pulse. Alternative preferred conditions when pulses of different wavelengths are selected are as follows: each of said first pulses has a wavelength within the range of 180 to 295 nm, a duration of from $1 \times 10^{-5}$ to $1 \times 10^{-10}$ seconds, and a flux of $1 \times 10^{13}$ to $5 \times 10^{14}$ photons/cm², and each second pulse has a wavelength within the range of 300 to 450 nm, a duration in the range of from $5 \times 10^{-9}$ to $1 \times 10^{-12}$ seconds, and a flux of $1 \times 10^{15}$ to $1 \times 10^{18}$ photons/cm², and each second pulse is applied within $1 \times 10^{-6}$ seconds of each first pulse.

Further embodiments of the present invention relate to methods of preparing killed virus vaccine, and to vaccines so prepared. These include the irradiation of a virus which is comprised of a nucleic acid portion and a tryptophan-containing protein coat. The subject irradiation is performed using ultra short high intensity laser pulses of different wavelengths to cause the non-linear photolysis of the nucleic acid components of the virus while leaving their surrounding protein coats substantially intact.

More specifically, these embodiments involve a process of preparing a killed virus vaccine, comprising the steps of (a) providing a virus containing solution, said virus comprising a nucleic acid portion and a tryptophan-containing protein coat; (b) irradiating a target region of a thin layer of said virus containing solution with one or more first light pulse(s) of wavelength(s) within a first wavelength range of 220 to 280 nm, said first pulse(s) each having a duration of less than $2 \times 10^{-8}$ seconds per pulse, and having a combined flux within said first wavelength range of between about $1 \times 10^{13}$ to $1 \times 10^{16}$ photons per cm²; and (c) irradiating said target region of said layer with one or more second higher intensity light pulse(s) of wavelength(s) within a second wavelength range above about 350 nm, said second pulse(s) each having a duration of less than $2 \times 10^{-8}$ seconds, and each having a flux within said second wavelength range of at least about $1 \times 10^{15}$ photons per cm², each of said second pulse(s) being applied to said layer within up to one microsecond after each of said first pulse(s), whereby said nucleic acid portion of said virus is inactivated while alteration of the structure of said protein coat is minimized. Preferred conditions for this embodiment are as follows: said combined flux within said first wavelength range is between about $1 \times 10^{14}$ and $5 \times 10^{14}$ photons/cm²; each of said second pulses within said second wavelength range has a flux of about $1 \times 10^{17}$ to $1 \times 10^{18}$ photons/cm²; said first pulses and said second pulses each have a duration of between about $9 \times 10^{-10}$ and $1 \times 10^{-12}$ seconds; said second wavelength range is 360 to 410 nm or 500 to 560 nm; said first pulses are applied at a frequency of between 10 and 1 000 000 pulses per second; said first and second pulses are applied substantially simultaneously; said layer has a thickness of less than 0,5 mm; said layer has a thickness of about 0,2 mm, said fraction is flowed through said target region at a rate which exposes each portion thereof to said combined fluxe   e.g., flowed across each millimeter of target region at a rate of about 5 ml/second; and said light pulses are laser pulses.

Further preferred embodiments of the present invention relate to processes for producing tryptophan-containing proteins, and to proteins so produced. They comprise culturing cells of mammalian origin to produce those proteins, causing or permitting those cells to release those proteins into a harvest medium and inactivating nucleic acid components in that medium by exposing that medium to a plurality of pulses of high intensity laser light of different wavelengths which are differentially absorbed by those nucleic acid components in their ground and excited states. Using this process, nulceic acid inactive, protein rich end products are produced.

More specifically, this embodiment can involve a process for producing tryptophan-containing proteins, comprising the steps of (a) providing cells of mammalian origin which, when cultured, produce said tryptophan-containing proteins; (b) culturing said cells; (c) releasing said proteins into a medium; and (d) inactivating the nucleic acid components in said medium by exposing said medium to a plurality of pulses of high intensity laser light of different wavelengths which are differentially absorbed by said nucleic acid components in their ground and excited states to produce nucleic acid inactive, protein rich end products.

Preferably, said inactivating step further comprises irradiating said medium with a first light pulse of a first wavelength of sufficient flux to raise a portion of said nucleic acid components from their ground state to an excited state, yet not sufficient to inactivate the proteins in said solution. More preferably, said inactivating step further comprises irradiating said nucleic acids while in said excited state with a second light pulse which is absorbed by nucleic acids in said excited state, but not substantially by said proteins in their ground state, to raise said acids to higher energy states to thereby cause photolysis of said nucleic acids while minimizing the photolysis of said proteins.

Preferred conditions for this embodiment are as follows:
said second pulse is applied during the triplet lifetime
of said portion of said nucleic acids, or said second
light pulse is applied within 1 picosecond after said
first light pulse, or said first and second pulses are
simultaneously applied; the wavelength of said first pulse
is between 220 and 280 nm; the duration of said first and
said second pulse is less than $2 \times 10^{-8}$ seconds, preferab-
ly the duration of said first and said second pulse is bet-
ween about $1 \times 10^{-12}$ and $9 \times 10^{-10}$ seconds; said first pulse
has a flux of less than about $5 \times 10^{14}$ photons/cm², pre-
ferably a flux of between about $1 \times 10^{13}$ and $5 \times 10^{14}$ photons
per cm², and more preferably a flux of about $1 \times 10^{14}$ to
$5 \times 10^{14}$ photons/cm²; said second pulse has a wavelength
above about 350 nm, preferably a wavelength of between
about 350 and 410 nm, or between about 500 and 560 nm;
said second pulse has a flux of about $1 \times 10^{15}$ to $1 \times 10^{18}$
photons/cm², preferably a flux of about $1 \times 10^{17}$ photons
per cm²; said light pulses are pulses of laser light; and
said light pulses are applied by a single laser.

As noted, the process of the invention is preferably
applicable to such biological fluids as blood sera or
blood fractions; media of mammalian cell origin containing
proteinaceous products and a nucleic acid component; and
viruses having tryptophan-containing protein coats. Those
of ordinary skill in the art will recognize, however, that
each of these embodiments will prefer a different degree
of nucleic acid inactivation and will tolerate a different degree of protein analysis. Since killed virus
vaccines may have some live virus, and need not retain all
of the original protein coat intact, it is anticipated
that reductions in nucleic acid or viral activity in this
application can be as low as $10^4$, preferably $10^6$, and
photolysis of viral protein coats as high as about 40%,
preferably 20%. By contrast, in blood and mammalian cell product applications, nucleic acid or viral activity reductions of at least $10^6$, preferably $10^8$, are sometimes desired. In blood or in applications involving the production of pharmaceutical products (such as insulin or other physiologic proteins), protein inactivation should not exceed 35%, preferably 20%, more preferably 5%, and most preferably be less than 2%. Where proteinaceous products are intended for non-pharmaceutical uses, lower protein yields can be accepted in order to optimize other process parameters.

Additional embodiments of the invention are illustrated in the following examples which are understood to be simulated and prophetical rather than as representations of work actually done.

EXAMPLE 1

This example illustrates the application of an embodiment of the invention to sterilize a biological medium comprising human plasma. The protein activity of the plasma can be assayed by a standard method such as the Partial Thromboplastin Time (PTT), a measure of the ability of the plasma proteins to form a clot. An increase of 2 sec. in the PTT corresponds to approximately a 10% decrease in the activity of the plasma proteins. The plasma, for purposes of this example, is deliberately infected with a mammalian virus, Simian Virus 40 (SV40), an easily titered virus of approximately the same size as hepatitis A. A sample of the plasma is flowed through a quartz tube 0,5 mm square. The rate of flow is controlled by a pump at a rate of $3 \times 10^{-4}$ ml/sec. thus establishing a flow velocity of $1,2 \times 10^{-1}$ cm/sec. through the target region. A Q-switched Nd:YAG laser is operated at a repetition rate of 20 Hz and produces pulses of $5 \times 10^{-9}$ sec. duration. The technique of harmonic generation is used to produce two pulses from the original pulse: a first pulse of a wavelength of 266 nm and a second pulse of a wavelength of 353 nm. The pulse at 266 nm is adjusted to contain $2 \times 10^{11}$ photons and

the pulse at 353 nm is adjusted to contain $1,2 \times 10^{15}$ photons. The pulses are focussed by a lens to a spot size of $4 \times 10^{-3}$ cm², producing a flux of $5 \times 10^{13}$ photons per cm² at 266 nm and $3 \times 10^{17}$ photons/cm² at 353 nm. The pulses arrive at the sample substantially simultaneously. Under these conditions, the average volume element of the plasma sample has a residence time in the target region of 0,5 seconds and, therefore, receives 10 repetitions of each pulse. The combined flux at 266 nm experienced by the average volume element is $5 \times 10^{14}$ photons/cm². After the sample has been processed as described, it is assayed for both viral activity and protein activity. It is found that the viral activity, as measured by the titer of SV40, has been reduced by a factor of $10^{6}$, and the protein activity has remained at 90% of its original value.

## EXAMPLE 2

This example illustrates the application of an embodiment of the invention in which pulses of the same wavelength are used to sterilize a biological medium comprising human plasma. The plasma, for purposes of this example, is deliberately infected with bacteriophage T4 which can be titered by a plaque-forming assay on an E.Coli. host. The protein activity is measured by the PTT (see Example 1). A sample of the plasma is flowed through a quartz tube with a cross-section of $2 \times 0,05$ cm. The laser beam enters through the 2cm face to result in an optical path length of 0,05 cm. A pump controls the flow rate of 1 ml/sec., establishing a flow velocity of 10 cm/sec. through the target region. An excimer laser is operated to produce pulses at 258 nm with a repetition rate of 200 Hz. Each pulse has a duration of $1 \times 10^{-8}$ sec. and contains $1 \times 10^{17}$ photons. These pulses are passed through a cylindrical lens and onto the target region, illuminating a target area of $2 \times 0,5$ cm. At the flow rate of 1 ml/sec. the average volume element requires 0,05 sec. to traverse the target region and receives 10 laser pulses. The flux from each

pulse is $1 \times 10^{17}$ photons/cm² and the total flux experienced by each volume element is $1 \times 10^{18}$ photons/cm². Under these conditions, the nucleic activity of the plasma sample, as assayed by the T4 titer, is reduced by a factor of $1 \times 10^{6}$, while the protein acitivity has remained at 65% of its original value.

## EXAMPLE 3

Example 2 is repeated except that the excimer laser is modified to produce pulses of $5 \times 10^{-12}$ seconds duration with $5 \times 10^{15}$ photons in a pulse at 258 nm. The repetition rate remains 200 Hz. The sample of the T4 in human plasma is flowed through a quartz tube of cross-section of $0,5 \times 0,05$ cm and a pump regulates the flow at 0,5 ml/sec., establishing a flow velocity of 20 cm/sec. through the target region. The laser pulses are passed through a cylindrical lens to achieve a target region of $0,1 \times 0,5$ cm. At the flow rate of 0,5 ml/sec. the average volume element spends $5 \times 10^{-3}$ seconds in the target region and receives only one pulse from the laser. The flux of this pulse is $1 \times 10^{17}$ photons/cm². Under these conditions the nucleic acid acitivity of the plasma sample is reduced by a factor of $1 \times 10^{6}$ from its original value while at least 90% of the original protein activity is preserved.

## EXAMPLE 4

This example illustrates the application of an embodiment of this invention to sterilize human blood fraction Factor VIII. The activity of Factor VIII can be accurately measured by colorimetric methods commercially available in kit form. A sample of lyophilized Factor VIII is reconstituted according to the packaged instructions and, for the purpose of this example, is deliberately infected with bacteriophage T7. The titer of T7 is obtained by a plaque-forming assay on an E.Coli.host. The sample is flowed through a quartz tube of cross-section $0,1 \times 0,05$ cm. The laser pulses strike the 0,1 cm face and thus the optical path length is 0,05 cm. A pump establishes the flow rate at $2 \times 10^{-3}$ ml/sec. A passively mode-locked Nd:YAG laser operates at a repetition rate of 20 Hz. The pulses have a time duration of $20 \times 10^{-12}$ sec. and pulses at 532 nm and 266 nm are produced by harmonic generation. The pulse at 532 nm is adjusted to contain $5 \times 10^{15}$ photons and the pulse at 266 nm is adjusted to contain $1 \times 10^{11}$ photons. By an arrangement of mirrors and lenses the pulses are made to arrive at the target region with the peak of the 266 nm pulse $1 \times 10^{-12}$ seconds before the peak of the 532 nm pulse. Both pulses illuminate a cross-sectional area of $5 \times 10^{-3}$ cm². The average volume element of the sample is struck by 5 repetitions of each pulse, i.e., the 266 nm pulse and the 532 nm pulse. The flux of each 266 nm pulse is $2 \times 10^{13}$ photons/cm², and the flux of each 532 nm pulse is $1 \times 10^{18}$ photons/cm². The average volume element in the sample receives a combined flux of $1 \times 10^{14}$ photons/cm² at 266 nm. The sample is analyzed after treatment and it is found that the nucleic acid activity, as measured by the titer of T7, is reduced by a factor of $1 \times 10^{6}$ while the protein activity remains at 98% of its value before irradiation.

## EXAMPLES 5 - 11

Example 4 is repeated with all sample parameters (i.e., flow rate, target region, quartz tube cross-section) unchanged except the Nd:YAG laser is now used to run a system of dye lasers producing output pulses that are variable in wavelength. Each time the Nd-YAG laser fires, two dye laser pulses having duration of $5 \times 10^{-12}$ seconds each are simultaneously produced. The results of the treatment in this series of examples are presented in tabular form.

| EXAMPLE No. | Wavelength of 1st Pulse (Flux = $2 \times 10^{13}$ photons/cm² per pulse) | Wavelength of 2nd Pulse (Flux = $2 \times 10^{18}$ photons/cm² per pulse) |
|---|---|---|
| 5 | 260 | 530 |
| 6 | 270 | 530 |
| 7 | 290 | 530 |
| 8 | 240 | 530 |
| 9 | 260 | 400 |
| 10 | 260 | 600 |
| 11 | 260 | 700 |

| EXAMPLE No. | Fraction of Original Titer of T7 | Percent of Original Protein Activity |
|---|---|---|
| 5 | $5 \times 10^{-7}$ | 98 % |
| 6 | $1 \times 10^{-6}$ | 98 % |
| 7 | $1 \times 10^{-1}$ | 98 % |
| 8 | $1 \times 10^{-3}$ | 99 % |
| 9 | $1 \times 10^{-5}$ | 98 % |
| 10 | $1 \times 10^{-4}$ | 98 % |
| 11 | $1 \times 10^{-3}$ | 98 % |

EXAMPLE 12

This example illustrates an embodiment of the invention in the treatment of a biological medium comprising human whole blood. The blood is, for purposes of this example, deliberately infected with bacteriophage T4 and the activity of the clotting proteins is measured using the PTT. The oxygen affinity of the hemoglobin proteins is monitored by standard methods. A laser system is used to produce pulses of $1 \times 10^{-13}$ seconds duration. Each pulse has a flux of $5 \times 10^{15}$ photons at a wavelength of 260 nm. The repetition rate is 200 Hz. The sample of T4 human blood is flowed through a quartz tube of cross-section 0,5 x 0,5 cm and a pump regulates the flow at 0,5 ml/cm, establishing a flow velocity of 20 cm/sec. through the target region. The laser pulses are passed through a cylindrical lens to achieve a target region of 0,1 x 0,5 cm.

The flux of each pulse at the target region is thus $1 \times 10^{17}$ photons/cm². Pulses of this duration and intensity are found to effectively penetrate ("bleach") red blood cells. Results indicate that nucleic acid activity, as measured by the titer of T4, is reduced by a factor of $1 \times 10^{6}$, while 90% of the original clotting protein activity is maintained and the oxygen affinity of the hemoglobin proteins shows no observable decrease.

In sum, the present invention provides a generic process for sterilization of biological media with a wide variety of specific embodiments. In these embodiments, pulses of light, preferably intense laser light, are used to selectively photolyze DNA- or RNA-containing nucleic acids in the presence of proteins. This selectivity is achieved by the use of pulses whose wavelength, time duration, time spacing and intensity are under the control of the laser operator, following the teachings herein.

From the above, those of ordinary skill in this art will recognize the applicability of this process to selectively photolyze the nucleic acid components in preference to and in the presence of proteins. Those of ordinary skill in this art will further recognize that conventional laser crystals, electronics and optics can be used to readily practice the described process of this invention. Those of ordinary skill in the art will also recognize that the exact laser power, wavelength, optimum sample handling, and so on may be varied somewhat in view of the foregoing description to achieve optimum results without departing from the scope of the present invention, which is described more particularly in the appended claims.

- 40 -

CLAIMS:

1. Process for treating a biological medium to photolyze nucleic acids in preference to proteins present therein, characterized in that it comprises irradiating said medium with pulsed light of wavelength and flux selected so that (1) the nucleic acids in their ground state absorb radiation and thereby rise to an excited state or states, (2) the nucleic acids in their excited states absorb radiation and thereby rise to higher energy states and undergo photolysis, and (3) the proteins in their ground or their excited states do not absorb sufficient radiation to undergo substantial photolysis.

2. The process of claim 1, characterized in that the biological medium is a solution selected from blood, blood fractions, genetically engineered proteinacous products of mammalian cells, and vaccine preparations containing viral DNA or RNA in a protein coat.

3. The process of claim 2, characterized in that the photolysis reduces nucleic acid activity by at least $10^4$ and reduces protein functionality by no more than 40%.

4. The process of claim 3, characterized in that the biological medium is selected from whole blood, blood plasma, blood serum, blood factor VIII, and blood factor XI comprising nucleic acids in the form of DNA- or RNA-containing pathogens, genetically engineered proteinacous products of mammalian cells comprising nucleic acids in the form of active mammalian DNA or RNA, and vaccine preparations comprising nucleic acids in the form of viral DNA or RNA in a protein coat; and that the photolysis reduces nucleic acid activity (viral activity) by at least $10^4$, and preferably by at least $10^6$, and reduces functionality of the protein coat by no more than 40%, and preferably by no more than 35%.

5.      The process of claim 4, characterized in that
the biological medium is an aqueous solution comprising
tryptophan-containing proteins; and that the photolysis
reduces substantially all of the nucleic acid activity
and reduces substantially none of the protein functionality.

6.      The process of claim 1 for treating a biological
medium containing nucleic acids and proteins, characterized in that it comprises irradiating said medium with a
plurality of laser pulses of wavelength and intensity selected so that said nucleic acids are photolyzed in preference to said proteins

7.      The process of claim 1 or 6, characterized in
that the pulsed light is comprised of laser pulses having
substantially the same wavelength.

8.      The process of claim 7, characterized in that
each of said pulses has a wavelength within the range of
180 to 295 nm, and preferably of 220 to 280 nm, a duration
less than $1 \times 10^{-5}$ seconds, and preferably in the range of
$5 \times 10^{-9}$ to $1 \times 10^{-15}$ seconds, and most preferably of
$1 \times 10^{-12}$ seconds, and a flux greater than $1 \times 10^{15}$ photons
per cm², and preferably in the range of from $1 \times 10^{5}$ to
$1 \times 10^{18}$, and most preferably in the range of from $1 \times 10^{17}$
to $1 \times 10^{18}$ photons per cm².

9.      The process of claim 1, characterized in that
the pulsed light is comprised of laser pulses having
different wavelengths.

10.      The process of claim 1 or 9, characterized in that
said laser pulses comprise one or more pulse(s) of a first
wavelength and flux sufficient to raise a substantial
portion of said nucleic acids from their ground state to
an excited state, yet not sufficient to photolyze a substantial portion of said proteins.

11.      The process of claim 10, characterized in that
said laser pulses further comprise a second pulse which
is preferentially absorbed by the nulceic acids in said
excited state, but not substantially by said proteins,
to thereby cause photolysis of said nucleic acids while
minimizing photolysis of said proteins.

12.      The process of claims 9, 10 or 11, characterized
in  that said laser pulses comprise one or more first
pulse(s) of wavelength and flux selected so that said
nucleic acids in their ground state absorb radiation and
thereby rise to an excited state or states, one or more
additional pulse(s) (second pulses) of wavelength and flux
selected so that said nucleic acids in their excited state
or states absorb radiation and thereby undergo photolysis,
and said proteins do not absorb sufficient radiation from
any of the pulses to undergo substantial photolysis.

13.      The process of claim 12, characterized in that
said excited states of nucleic acids are selected from
the singlet state and the triplet state, and that said
additional pulse or pulses are applied during the lifetime
of said excited states of nucleic acids.

14.      The process of claim 12, characterized in that
said first and second pulses are simultaneously or sequentially applied.

15.      The process of claim 12, characterized in that
each of said second pulses is applied within 1 microsecond
after each of said first pulses, and said first and second
pulses are applied in alternating sequences of one or more
than one pulse each.

16.      The process of claim 12, characterized in that each of said first pulses has a wavelength within the range of 180 to 350 nm, preferably of 180 to 295 nm, a duration of less than $1 \times 10^{-5}$ seconds, and preferably in the range of $1 \times 10^{-10}$ to $10^{-14}$ seconds, and most preferably in the range of $10^{-5}$ to $10^{-10}$ seconds, and a flux less than $1 \times 10^{18}$, and preferably within the range of $1 \times 10^{13}$ to $5 \times 10^{14}$ photons per cm²; and that each of said second pulses has a wavelength within the range of 300 to 700 nm, preferably of 500 to 560 nm, or of 300 to 450 nm, a duration of less than $1 \times 10^{-5}$ seconds, and preferably within the range of $1 \times 10^{-10}$ to $1 \times 10^{-12}$ seconds, and most preferably of $5 \times 10^{-9}$ to $1 \times 10^{-12}$ seconds, and a flux of greater than $1 \times 10^{15}$, and preferably not exceeding $1 \times 10^{18}$ photons per cm².

17.    The process of claim 16, characterized in that each second pulse is applied within $3 \times 10^{-12}$ seconds of each first pulse.

18.    The process of claim 16, characterized in that each second pulse is applied within $1 \times 10^{-6}$ seconds of each first pulse.

19.    A process for sterilizing a biological fluid comprising proteins, which fluid is suspected of carrying DNA- or RNA-containing pathogens, characterized in that it comprises the steps of (a) irradiating said fluid with one or more first light pulse(s) of wavelength(s) within a first wavelenth range of 220 to 280 nm, said first pulse(s) each having a duration of less than $2 \times 10^{-8}$ seconds, and preferably between $9 \times 10^{-9}$ and $1 \times 10^{-12}$ seconds per pulse; and having a combined flux within said first wavelength range of between about $1 \times 10^{13}$ to $1 \times 10^{16}$, and preferably between $1 \times 10^{14}$ and $5 \times 10^{14}$ photons per cm²; and (b) irradiating said fluid with one or more second higher intensity light pulse(s) of wavelength(s) within a second wavelength range above 300 nm, preferably from 350 to 410 or 510 to 560 nm, said second pulse(s) each having a duration of less than $2 \times 10^{-8}$ seconds, and preferably between $9 \times 10^{-10}$ and $1 \times 10^{-12}$ seconds, and each having a flux within said second wavelength range of at least about $1 \times 10^{15}$, and preferably of $1 \times 10^{17}$ to $1 \times 10^{18}$ photons per cm², each of said second pulse(s) being applied to said fluid within up to one microsecond after each of said first pulse(s), whereby RNA or DNA-containing pathogens in the fluid are substantially inactivated while proteins in the fluid are substantially unaltered.

20.     The process of claim 19, characterized in that said first pulse(s) is (are) applied at a frequency of between 10 and 1.000.000 pulses per second.

21.     The process of claim 19, characterized in that said first and second pulses are applied substantially simultaneously.

22.     The process of claim 19, characterized in that said first and second pulses are applied in alternating sequences of one or more than one pulse each.

23.     The process of claim 19, characterized in that it comprises placing said fluid in a target region of a thin layer and irradiating said target region with said pulses.

24.     The process of claim 23, characterized in that said layer has a  thickness of less than 0,5 mm, and preferably of 0,2 mm.

25.     The process of claim 23, characterized in that said fluid is passed through said target region at a rate which exposes each portion thereof to said combined fluxes.

26.     The process of claim 25, characterized in that said fluid is passed through each millimeter of said target region at a rate of about 5 milliliters per second.

27.     The process of claim 19, characterized in that said light pulses are laser pulses.

28.     The process of claim 27, characterized in that said light pulses are pulses from a single laser.

29.     The process of claim 27, characterized in that the fluid is selected from the group consisting of blood, blood fractions such as blood plasma, blood factors or blood serum, genetically engineered proteinaceous products of mammalian cells, and vaccine intermediates containing viral DNA or RNA in a protein coat.

30.     The process of claim 29, characterized in that said pulses are applied from a plurality of directions to strike substantially all of the plasma and serum present in said fluid.

31.     The process of claims 1 to 4 for preparing a killed virus vaccine, characterized that it comprises the steps of (a) providing a virus containing solution, said virus comprising a nucleic acid portion and a tryptophan-containing protein coat; (b) irradiating a target region of a thin layer of said virus containing solution with one or more first light pulse(s) of wavelength(s) within a first wavelength range of 220 to 280 nm, said first pulse(s) each having a duration of less than $2 \times 10^{-8}$ seconds per pulse; and having a combined flux within said first wavelength range of between about $1 \times 10^{13}$ to $1 \times 10^{16}$ photons per cm²; and (c) irradiating said target region of said layer with one or more second higher intensity light pulse(s) of wavelength(s) within a second wavelength range above about 350 nm, said second pulse(s) each having a duration of less than $2 \times 10^{-8}$ seconds, and each having a flux within said second wavelength range of at least about $1 \times 10^{15}$ photons per cm², each of said second pulse(s) being applied to said layer within up to one microsecond after each of said first pulse(s), whereby said nucleic portion of said virus is inactivated while alteration of the structure of said protein coat is minimized.

32. A method of producing tryptophan-containing proteins, characterized in that it comprises the steps of (a) providing cells of mammalian origin which, when cultured, produce said tryptophan-containing proteins; (b) culturing said cells; (c) releasing said protein into a medium; and (d) inactivating the nulceic acid components in said medium by exposing it to a plurality of pulses of high intensity laser light of different wavelengths which are differentially absorbed by said nucleic acid components in their ground and excited states to produce nucleic acid inactive, protein rich end products.

33. The method of claim 32, characterized in that said inactivating step further comprises irradiating said medium with a first light pulse of a first wavelength of sufficient flux to raise a portion of said nucleic acid components from their ground state to an excited state, yet not sufficient to inactivate the proteins in said medium.

34. The method of claim 33, characterized in that said inactivating step further comprises irradiating said nucleic acids while in said excited state with a second light pulse which is absorbed by nucleic acids in said excited state, but not substantially by said proteins in their ground state, to raise said acids to higher energy states to thereby cause photolysis of said nucleic acids

0152072

while minimizing the photolysis of said proteins.

35. A biological medium treated by the process of one or more of claims 1 to 18.

36. A biological fluid sterilized by the process of one or more of claims 19 to 30.

37. A blood fraction or blood sterilized by the process of claim 29 or 30.

38. A virus vaccine prepared by the process of one or more of claims 1 to 18 and 31 to 34.

39. A protein composition prepared by the method of one or more of claims 1 to 34.

40. A blood fraction comprising plasma proteins prepared by the process of claims 1 to 30.

# CASE 1. SINGLE WAVELENGTH RADIATION BY CLASSICAL SOURCE

# CASE 2. TWO WAVELENGTH, TWO PULSE LASER IRRADIATION

ADDITIONAL STATES

SECOND PULSE
(MAY ALSO EXCITE
SINGLET)

HIGHER ENERGY STATES

{ H G F E D }

MULTIPLE PHOTO-
CHEMICAL PATHWAYS
NOW OCCUR

SECOND PULSE
(EXCITES TRIPLET)

OVERALL Q IS
SUM OF Q's

B       SINGLET

C   TRIPLET

SECOND PULSE
(NOT ABSORBED BY GROUND STATE)

FIRST PULSE

*Fig. 2*

A       GROUND